# EUROPÄISCHE PATENTSCHRIFT

(11) **EP 1 158 944 B1**
(45) Veröffentlichungstag und Bekanntmachung des Hinweises auf die Patenterteilung: **15.01.2003**
(21) Anmeldenummer: 99959347.8
(22) Anmeldetag: 26.11.1999
(51) Int. Cl.: A61F 13/15

(54) **HYGIENEARTIKEL MIT EINER EINZELUMVERPACKUNG**
HYGIENE ARTICLE HAVING AN INDIVIDUAL WRAPPING
ARTICLE D'HYGIENE POURVU D'UN EMBALLAGE INDIVIDUEL

(30) Priorität: 28.01.1999 DE 19903285
(43) Veröffentlichungstag der Anmeldung: 05.12.2001
(73) Patentinhaber: Paul Hartmann Aktiengesellschaft, 89522 Heidenheim (DE)
(72) Erfinder: GAUSE, Enno, D-89522 Heidenheim (DE)
(74) Vertreter: Friz, Oliver
(86) Internationale Anmeldenummer: EP9909180
(87) Internationale Veröffentlichungsnummer: WO00044325

(56) Entgegenhaltungen:
- EP-A- 0 750 896
- FR-A- 2 581 619
- GB-A- 2 060 398
- US-A- 4 564 108
- US-A- 4 605 403

## Beschreibung

Die Erfindung betrifft einen Hygieneartikel, wie eine Inkontinenzeinlage, Slipeinlage oder eine Damenbinde, mit einer Einzelumverpackung.

Hygieneartikel mit einer Einzelumverpackung sind aus einer großen Anzahl von Druckschriften bekannt. Die US-A-4,556,146 beschreibt eine Damenbinde, die auf eine flächenmäßig größere Hüllschicht aus einem flüssigkeitsundurchlässigen Material aufgelegt und zusammen mit der Hüllschicht um zwei senkrecht zu den Längsachsen verlaufende Querachsen auf sich selbst gefaltet ist, wodurch die Hüllschicht eine Einzelumverpackung für den Hygieneartikel bildet. Die auf sich selbst gefalteten Längsrandbereiche der Hüllschicht sind lösbar miteinander verbunden.

Es ist schwer möglich, einen gebrauchten Hygieneartikel derart mit der Hüllschicht zu umgeben, dass keine Flüssigkeit austreten kann und auch keine Geruchsbelästigung auftritt.

Aus der US 5,474,818 ist ein Hygieneartikel bekannt, der auf ähnliche Weise mit einer Einzelumverpackung versehen ist. Der Hygieneartikel selbst wird hierbei jedoch nicht gefaltet. Auf diese Weise ist die jeweilige Verbrauchslänge einer die Umhüllung bildenden Bahn sehr groß.

Die US-A-5,462,166 offenbart eine vergleichbare Einzelumverpackung, wobei im Überlappungsbereich der Längsenden der Umhüllung eine Klebelasche vorgesehen ist, um den gebrauchten Artikel in zusammengefalteter Konfiguration mit der Umhüllung zu halten. Das Problem des seitlichen Austretens von Flüssigkeit und die Geruchsbelästigung erweisen sich als nachteilig.

Aus der US-A-4,735,316 ist ein Hygieneartikel mit einer Einzelumverpackung bekannt, die aus einer Hüllschicht gebildet ist, auf welche der Hygieneartikel aufgelegt ist und die dann um die Längskanten des Hygieneartikels auf dessen Oberseite gefaltet ist. Die Hüllschicht weist an der körperabgewandten Seite des Hygieneartikels eine Z-förmige von den Längsseiten her in Querrichtung eingebrachte Faltung mit einer inneren und einer äußeren in Längsrichtung verlaufenden Falzlinie auf. Hierdurch soll zusätzliches Volumen zur Aufnahme des gebrauchten Hygieneartikels in das Innere der durch die Hüllschicht gebildeten Tasche geschaffen werden. Die Hüllschicht bildet daher einen Längsabschnitt einer schlauchförmige Bahn, in deren Innerem der Hygieneartikel vor dem Gebrauch aufgenommen ist. Eine platzsparende Faltung des Hygieneartikels zusammen mit der Einzelumverpackung ist nicht vorgesehen oder angeregt.

In der US-A-4,605,403 bildet ein Längsabschnitt einer parallel zu ihrer Längsachse Z-förmig gefalteten Schlauchbahn sowohl eine Abdeckung für einen Haftklebebereich an der körperabgewandten Seite einer Damenbinde als auch eine Entsorgungstasche für die gebrauchte Damenbinde. Eine Einzelumverpackung der Damenbinde vor dem Gebrauch ist nicht vorgesehen.

Eine einzelumverpackte Damenbinde mit den Merkmalen des Oberbegriffs des Anspruchs 1 ist aus FR-A-2 581 619 bekannt.

Aus GB-A-2 060 398 ist es bekannt, eine schlauchförmige Bahn als Einzelumverpackung für den gebrauchten Artikel in ebene Konfiguration zu falten und einer Damenbinde lösbar anzufügen.

EP-A-0 750 896 zeigt zwar eine einzelumverpackte Damenbinde, die auf eine eine Innenseite der Einzelumverpackung bildende Flachseite eines Längsabschnitts einer Bahn aufgelegt und zusammen mit dem Längsabschnitt gefaltet ist; eine schlauchförmige Bahn ist indessen nicht offenbart.

Der vorliegenden Erfindung liegt die Aufgabe zugrunde, einen Hygieneartikel mit einer Einzelumverpackung, welche vor dem ersten Gebrauch des Hygieneartikels einen Schutz während der Lagerhaltung sowie während des Transports und anschließenden Verkaufs des Hygieneartikels bis hin zur Ingebrauchnahme durch den Endverbraucher bildet, zu schaffen, der einerseits platzsparend mit verhältnismäßig geringer flächenhafter Ausdehnung und in hygienisch einwandfreiem vor Verschmutzung geschütztem Zustand, bspw. in einer Damenhandtasche mitgeführt werden kann und dessen Umverpackung andererseits geeignet ist, den gebrauchten Hygieneartikel sicher aufzunehmen, so dass keine Flüssigkeit und keine Gerüche aus dem mitgeführten Artikel austreten können, wobei ausgehend von der bekannten Damenbinde nach FR-A-2 581 619 ein anderes Produktionsverfahren geschaffen werden soll, das auch zu einer abweichenden einzelumverpackten Konfiguration führt.

Diese Aufgabe wird erfindungsgemäß gelöst durch einen Hygieneartikel mit einer Einzelumverpackung vor dem ersten Gebrauch, während der Lagerhaltung sowie während des Transports und anschließenden Verkaufs bis hin zur Ingebrauchnahme durch den Endverbraucher, mit den Merkmalen des Anspruchs 1.

Nach der Erfindung ist die Einzelumverpackung also aus einem Längsabschnitt der schlauchförmigen Bahn gebildet, auf dem der Hygieneartikel aufgebracht und sodann zusammen mit dem Längsabschnitt um Querachsen gefaltet ist, wodurch die Einzelumverpackung gebildet wird. Zum Fixieren des Hygieneartikels und der Einzelumverpackung in ihrer gefalteten Konfiguration sind die aufeinandergefalteten Längsrandbereiche des Längsabschnitts lösbar miteinander verbunden und zwar derart, dass sie in gefalteter Konfiguration vor dem ersten Gebrauch während der Lagerhaltung, des Transports und Verkaufs bis unmittelbar vor der Benutzung gehalten sind aber gleichwohl ein leichtes Öffnen also Lösen der aufeinandergefalteten Längsrandbereiche ermöglichen. Die lösbare Verbindung kann bspw. durch kohäsiv oder adhäsiv wirkende Klebematerialien oder durch Vorsehen einer Sollbruchlinie, vorzugsweise aber durch Thermoprägen verwirklicht werden. Der Längsabschnitt der schlauchförmigen Bahn bildet also zugleich die Einzelumverpackung als auch einen Entsorgungsbeutel für den Hygieneartikel, ohne dass der Hygieneartikel jedoch vor dem ersten Gebrauch im Inneren des Längsabschnitts aufgenommen wäre und auf umständliche und wenig benutzerfreundliche Weise aus dem Inneren der schlauchförmigen Umhüllung zum ersten Gebrauch entnommen werden müßte. Der Entsorgungsbeutel weist ein offenes und ein flüssigkeitsdicht verschlossenes Längsende auf, in welches der gebrauchte Hygieneartikel eingesteckt werden kann und darin flüssigkeitsdicht aufgenommen ist und bleibt.

Durch das Falten um zwei Querachsen wird ein sehr kompaktes Erscheinungsbild des in der Einzelumverpackung vorgesehenen Hygieneartikels erzielt und der Hygieneartikel kann auf platzsparende Weise, bspw. in einer Damenhandtasche, mitgeführt werden. Außerdem wird durch das Aufeinanderfalten auf zwei Querachsen im Unterschied bspw. zu der eingangs erwähnten US 5,474,818 Verpackungsmaterial eingespart.

Nach einer ganz besonders bevorzugten Ausführungsform des Hygieneartikels umfasst der Längsabschnitt der eben gefalteten schlauchförmigen Bahn zwei übereinander angeordnete die Flachseiten der Einzelumverpackung bildende Materialabschnitte, die in einem Abstand zu ihren jeweiligen Längsrändern in Längsrichtung flüssigkeitsdicht und unlösbar miteinander verbunden sind. Die beiden Materialabschnitte können ihrerseits von zwei übereinander zugeführten Flachmaterialbahnen gebildet sein, die durch flüssigkeitsdichtes und unlösbares Verbinden miteinander die schlauchförmige Bahn definieren. Aber auch wenn die schlauchförmige Bahn bereits einstückig zugeführt wird, erweist es sich als vorteilhaft wenn - wie vorstehend erwähnt - zu beiden Seiten und in einem Abstand zu den jeweiligen Längsrändern der eben gefalteten schlauchförmigen Bahn eine flüssigkeitsdichte und unlösbare Verbindung, vorzugsweise in Form einer Siegellinie vorgesehen wird, da solchenfalls in der Richtung außerhalb dieser flüssigkeitsdichten und unlösbaren Verbindung die lösbare Verbindung der aufeinandergefalteten Längsrandbereiche vorgesehen werden kann und es beim Öffnen der lösbaren Verbindung nicht zu Undichtigkeiten des Verpackungsbeutels kommen kann. Es wird also in weiterer Ausbildung der Erfindung vorgesehen, unlösbare Verbindungslinien, die jedoch nicht notwendigerweise geradlinig verlaufen müssen, sondern auch wellen- oder zickzackförmig erstreckt sein können, vorzusehen und in Querrichtung außerhalb dieser flüssigkeitdichten unlösbaren Verbindungslinien die lösbare Verbindung der aufeinandergefalteten Längsrandbereiche des Längsabschnitts der schlauchförmigen Bahn vorzusehen.

Nach einer weiteren besonders bevorzugten Ausführungsform der Erfindung weist wenigstens einer der eben aufeinandergefalteten Materialabschnitte an beiden Längsseiten eine Z-förmige Faltung mit einer inneren und einer äußeren in Längsrichtung verlaufenden Falzlinie auf. Durch diese weitere Maßnahme kann Aufnahmevolumen für den gebrauchten Hygieneartikel geschaffen werden, ohne dass die Einzelumverpackung eine wesentlich über die Breite des Hygieneartikels hinausgehende Abmessung aufweisen müßten.

Solchenfalls wird in weiterer Ausbildung dieses Erfindungsgedankens vorgeschlagen, dass die äußeren Falzlinien in Querrichtung innerhalb des Verlaufs der unlösbaren Verbindung der Materialabschnitte miteinander angeordnet sind. Dies erlaubt es, die unlösbaren Verbindungen der Materialabschnitte miteinander außerhalb der äußeren Falzlinien zu einem späteren Zeitpunkt als die Faltung vorzusehen, ohne dass die Z-förmige Faltung hiervon erfaßt oder beeinflußt wird. Schließlich soll die Z-förmige Faltung ja ein auffaltbares Aufnahmevolumen zur Verfügung stellen und nicht auf eine der Materialabschnitte unlösbar aufgesiegelt werden.

Gleichwohl ist auch eine Ausführungsform denkbar, bei der die äußeren Falzlinien nicht nach innen abgesetzt sind sondern bspw. randbündig mit den Längsrändern der Materialabschnitte verlaufen. Solchenfalls muß zum flüssigkeitsdichten unlösbaren Verbinden der eben gefalteten Materialabschnitte ein Trennblech oder dgl. in die Z-förmige Faltung eingeschoben werden oder es muß bei noch eingeschobenem Faltblech die unlösbare Verbindung der Materialabschnitte vorgesehen werden, um zu verhindern, dass die Faltung unlösbar gestellt wird. Um in diesem Fall zu verhindern, dass die Einzelumverpackung bzw. der hierdurch gebildete Entsorgungsbeutel im Bereich der Z-förmigen Faltung durch die lösbare Verbindung bzw. Trennung der aufeinandergefalteten Längsrandbereiche undicht wird, empfiehlt es sich, auch innerhalb der Z-förmigen Falte eine innere unlösbare Verbindung quasi als Dichtlinie vorzusehen. Eine derartige aufwendige Maßnahme erübrigt sich jedoch, wenn - wie vorstehend vorgeschlagen - die Z-förmige Faltung derart durchgeführt wird, dass die äußeren Falzlinien in Querrichtung innerhalb des Verlaufs der unlösbaren Verbindung der Materialabschnitte und damit auch innerhalb der aufeinandergefalteten Längsrandbereiche, die lösbar miteinander verbunden sind, verläuft.

Der Hygieneartikel umfaßt vorzugsweise an seiner körperabgewandten Außenseite, welche der die Innenseite bildenden Flachseite des Längsabschnitts der schlauchförmigen Bahn zugewandt ist, ein Haftklebemittel zum Befestigen des Artikels am Steg eines Unterbekleidungsstücks. Solchenfalls kann der Hygieneartikel über das Haftklebemittel direkt an der die Innenseite bildenden Flachseite anliegen, sofern er hiervon wieder ablösbar ist. Um die Ablösekraft des Hygieneartikels von der die Innenseite bildenden Flachseite der Einzelumverpackung erforderlichenfalls reduzieren zu können, kann eine Release-Beschichtung, z.B. in Form einer Silikonisierung, auf die Innenseite der Einzelumverpackung aufgebracht werden.

Nach einer weiteren Ausführungsvariante kann das Haftklebemittel auch über ein Ablösepapier an die Außenseite des Hygieneartikels appliziert sein. Solchenfalls liegt der Hygieneartikel unter Zwischenordnung des Ablösepapiers gegen die Innenseite der Einzelumverpackung an. Nach einer weiteren besonders bevorzugten Ausführungsform ist zwischen dem Ablösepapier und der die Innenseite bildenden Flachseite ein vorzugsweise haftklebendes Fixiermittel vorgesehen. Dies bringt den weiteren Vorteil mit sich, dass der Hygienieartikel samt Ablösepapier in vorbestimmter Stellung an der Innenseite gehalten werden kann, was das Falten des Hygieneartikels um die Querachsen erleichtert. Zum anderen können durch entsprechend gewählte Haftkraftunterschiede zwischen dem Ablösepapier und dem Haftklebemittel auf der körperabgewandten Seite des Hygieneartikels und dem vorzugsweise haftklebenden Fixiermittel zwischen Ablösepapier und der Innenseite der Einzelumverpackung erreicht werden, dass das Ablösepapier beim Entnehmen des Hygieneartikels zum Gebrauch durch die Wirkung des Fixiermittels an der Einzelumverpackung verbleibt. Der Benutzer braucht dann das Ablösepapier nicht separat zu entsorgen, mitzuführen oder wegzuwerfen, sondern es verbleibt an der Einzelumverpackung und wird mit dieser entweder gleich weggeworfen oder vorzugsweise aufbewahrt und nach Einbringen des gebrauchten Hygieneartikels mit diesem entsorgt.

Es ist an sich hinreichend, den Hygieneartikel in gefalteter Konfiguration in der Einzelumverpackung zu halten, indem nur die aufeinandergefalteten Längsrandbereiche des Längsabschnitts lösbar miteinander verbunden sind. Indessen kann es sich als vorteilhaft erweisen, wenn zusätzlich ein vorzugsweise haftklebendes Verschlussmittel vorgesehen ist, welches die um die beiden Querachsen aufeinandergefalteten Längsenden des Längsabschnitts der schlauchförmigen Bahn lösbar miteinander verbindet. Die Verschlussmittel können bspw. durch streifenförmige Laschen, die zusätzlich an ein Längsende des Längsabschnitts angefügt sind oder durch auf die Innenseite aufgebrachte Klebepunkte an sich beliebiger Geometrie gebildet sein.

Die vorliegende Erfindung betrifft auch ein Verfahren zum Herstellen eines Hygieneartikels mit einer Einzelumverpackung der vorstehend beschriebenen Art mit den Merkmalen des Anspruchs 13.

Zum Herstellen eines erfindungsgemäßen Hygieneartikels kann also entweder eine bspw. einstückig extrudierte schlauchförmige Bahn ohne Nahtstellen zugeführt werden, oder es kann eine oder eine erste und eine zweite Flachmaterialbahn zur Bildung der schlauchförmigen Bahn in Längsrichtung zugeführt werden. Im letzteren Fall werden die Flachmaterialbahnen in Längsrichtung beidseitig unlösbar miteinander verbunden, um die schlauchförmige Bahn zu bilden. Im ersten Fall werden aufeinandergefaltete Abschnitte der eben gefalteten schlauchförmigen Bahn ebenfalls beidseits in Längsrichtung und in einem Abstand zu ihren jeweiligen Längsrändern unlösbar miteinander verbunden. Auf diese Weise steht ein Längsrandbereich außerhalb der unlösbaren Verbindung zur Verfügung, um nach dem Falten um die Querachsen die aufeinandergefalteten Längsrandbereiche lösbar, bspw. durch Thermoprägen, miteinander zu verbinden.

Weitere Merkmale, Einzelheiten und Vorteile des erfindungsgemäßen Hygieneartikels mit Einzelumverpackung sowie des Verfahrens zu seiner Herstellung ergeben sich aus den beigefügten Ansprüchen, für deren Merkmale auch jeweils für sich alleingenommen Schutz beansprucht wird, und aus der zeichnerischen Darstellung und folgenden Beschreibung bevorzugter Ausführungsformen der Erfindung. In der Zeichnung zeigt:
- Figur 1: eine schematische Schnittdarstellung eines Hygieneartikels im auf eine schlauchförmige Bahn aufgelegten Zustand während der Herstellung;
- Figur 2: eine perspektivische Ansicht des Hygieneartikels im auf die schlauchförmige Bahn aufgelegten Zustand;
- Figur 3: eine perspektivische Ansicht der geschlossenen Einzelumverpackung mit darin enthaltenem, jedoch nicht dargestellten Hygieneartikel zur Lagerung, Transport oder Verkauf oder zur weiteren stapelweisen Verpackung;
- Fig.4a-4d: verschiedene Darstellungen des Hygieneartikels mit Einzelumverpackung während der Herstellung.

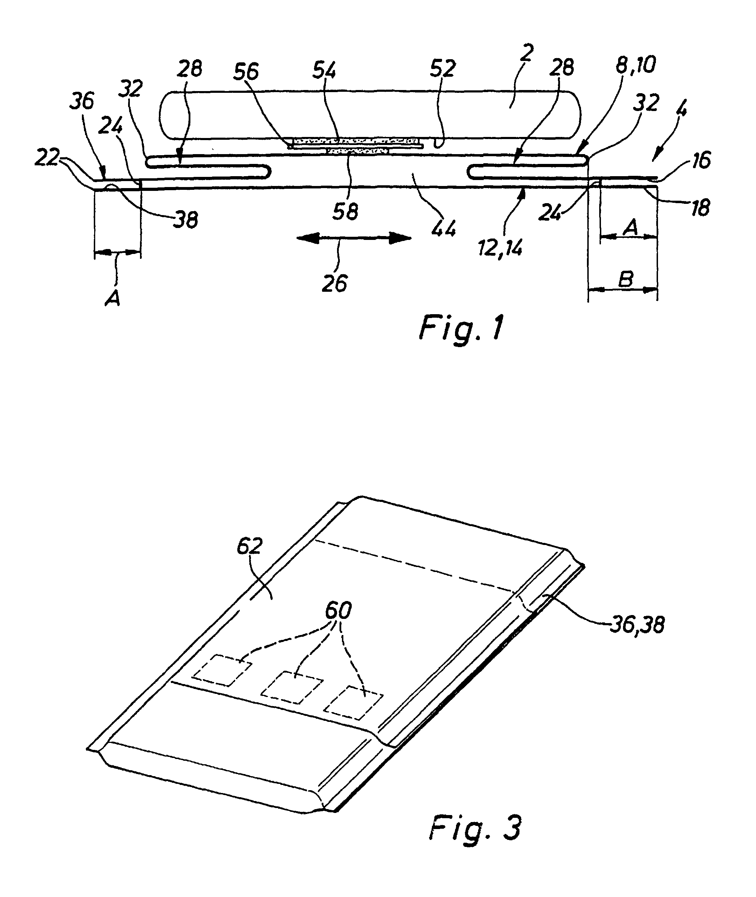

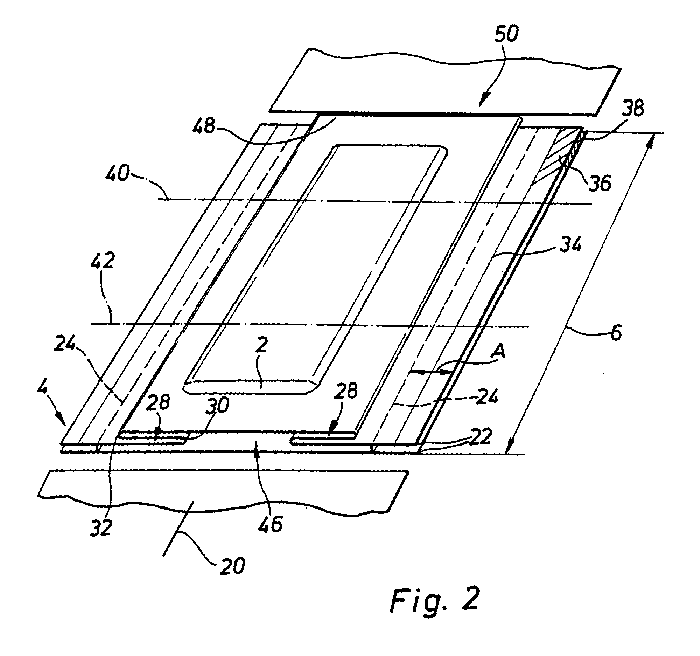

Die Figuren 1 und 2 zeigen einen, lediglich schematisch angedeuteten Hygieneartikel 2 in Form einer Inkontinenzeinlage, die am Steg eines Unterbekleidungsstücks befestigbar ist, im auf eine schlauchförmige Bahn 4 aufgelegten Zustand in einem Herstellungszwischenschritt.

Wie nachfolgend noch im einzelnen beschrieben werden wird, ist die Einzelumverpackung aus einem Längsabschnitt 6 der schlauchförmigen Bahn 4 gebildet. Der Längsabschnitt 6 der eben gefalteten schlauchförmigen Bahn 4 umfasst eine eine Innenseite 8 der Einzelumverpackung bildende Flachseite 10 und eine eine Außenseite 12 der Einzelumverpackung bildende Flachseite 14. Die Flachseiten 10, 14 sind die voneinander abgewandten Seiten von Materialabschnitten 16 und 18, welche die schlauchförmige Bahn 4 bilden. Die Materialabschnitte 16, 18 sind in Längsrichtung 20 und in einem Abstand A von den jeweiligen Längsrändern 22 flüssigkeitsdicht und unlösbar miteinander verbunden. Dies ist durch die als unterbrochene Linie angedeutete Verbindungslinie 24, die durch eine in Längsrichtung 20 verlaufende Siegelnaht gebildet ist, dargestellt.

Der in der Darstellung obere, dem Hygieneartikel 2 zugewandte Materialabschnitt 16 umfasst beidseits seiner Längsrichtung 20 eine in Querrichtung 26 eingebrachte Z-förmige Faltung 28 mit einer inneren Falzlinie 30 und einer äußeren Falzlinie 32. Die Z-förmige Faltung 28 wurde derart eingebracht, dass die äußeren Falzlinien 32 in Querrichtung 26 innerhalb der unlösbaren Verbindungslinien 24 der Materialabschnitte 16, 18 verlaufen. Sie haben also einen Abstand B von den Längsrändern 22, der größer ist als der Abstand A. Dies bringt den Vorteil mit sich, dass zuerst die Z-förmige Faltung 28 in die den oberen Materialabschnitt 16 bildenden Flachmaterialbahn eingebracht werden kann und im Anschluß hieran die unlösbare Verbindung 24 zwischen den Materialabschnitten 16 und 18 vorgesehen werden kann, die dann die Z-förmige Faltung 28 nicht erfasst, da sie in Querrichtung 26 außerhalb der äußeren Falzlinie 32 zu liegen kommt.

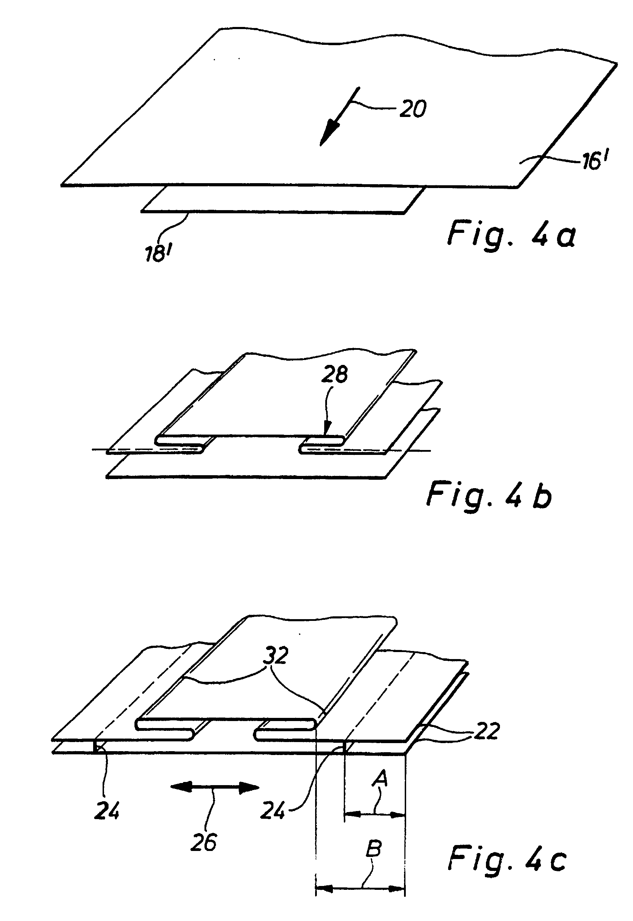

In Figur 2 ist durch eine lediglich gedachte Linie 34 ein Längsrandbereich 36 des oberen Materialabschnitts 16 sowie ein Längsrandbereich 38 des darunter angeordneten Materialabschnitts 18 bezeichnet. Diese Längsrandbereiche 36, 38 liegen außerhalb der jeweiligen äußeren Falzlinie 32 und außerhalb der jeweiligen Linie der unlösbaren flüssigkeitsdichten Verbindung 24. Wenn ein jeweiliger Längsabschnitt 6 nach dem Trennen von der schlauchförmigen Bahn 4 um zwei Querachsen 40, 42 zusammen mit dem Hygieneartikel 2 gefaltet wird, so werden Teilabschnitte der Längsrandbereiche 36, 38 auf sich selbst gefaltet, so wie dies in Figur 3 dargestellt ist. Zum Halten des Hygieneartikels samt seiner Einzelumverpackung in der in Figur 3 dargestellten gefalteten Konfiguration werden die aufeinandergefalteten Teilabschnitte der Längsrandbereiche 36, 38 lösbar miteinander verbunden, vorzugsweise durch Thermoprägen. Hierdurch werden die Längsrandbereiche teilweise von dem Prägewerkzeug durchdrungen. In jedem Fall kommt es beim Lösen der insbesondere rasterartig aufgebrachten lösbaren Verbindung zu Beschädigungen der Längsrandbereiche 36, 38 der Materialabschnitte 16, 18. Da die Längsrandbereiche 36, 38 aber außerhalb der äußeren Falzlinien 32 und der Linien der unlösbaren Verbindung 24 liegen ist hiervon die Dichtigkeit innerhalb der Linien der unlösbaren Verbindung 24 nicht betroffen. Der untere Materialabschnitt 18 und der obere Materialabschnitt 16 mit seiner Z-förmigen Faltung definieren daher ein flüssigkeitsdichtes Aufnahmevolumen 44 und mithin einen Entsorgungsbeutel für den Hygieneartikel nach seinem Gebrauch. Der gebrauchte Hygieneartikel 2 kann dann durch eine Öffnung 46 in das Innere des Aufnahmevolumens eingeführt werden. An dem gegenüberliegenden Ende ist eine flüssigkeitsundurchlässige Quernaht 48, die unmittelbar neben der Trennlinie 50 zum Abtrennen des Längsabschnitts 6 von der durchgehenden schlauchförmigen Bahn 4 angebracht ist.

Aus Figur 1 ist ferner ersichtlich, dass der Hygieneartikel 2 auf seiner körperabgewandten Seite 52 ein Haftklebemittel 54 aufweist, mit dem der Hygieneartikel 2 am Steg eines Unterbekleidungsstücks fixierbar ist. Das flächenhaft aufgetragene Haftklebemittel 54 ist von einem silikonisierten Ablösepapier 56 überdeckt und wird über dieses Ablösepapier auf die körperabgewandte Seite 52 des Hygieneartikels 2 appliziert. Zwischen dem Ablösepapier 56 und der Innenseite 8 ist ein haftklebendes Fixiermittel 58 vorgesehen. Die Haftkraftunterschiede zwischen dem Ablösepapier 56 und dem Haftklebemittel 54 bzw, dem haftklebenden Fixiermittel 58 sind derart, dass beim Ablösen des Hygieneartikels 2 von der Innenseite 8 der Einzelumverpackung das Ablösepapier 56 an der Innenseite 8 verbleibt. Es wird darauf hingewiesen, dass es ebenfalls möglich ist, das Ablösepapier 56 wegzulassen, so dass der Hygieneartikel 2 direkt über das Haftklebemittel 54 gegen die Innenseite 8 der Einzelumverpackung anliegt. Solchenfalls können die Haftkräfte zu der Innenseite 8 über eine zusätzliche Release-Beschichtung auf der Innenseite 8 der Einzelumverpackung, bspw. in Form einer Silikonisierung, auf die gewünschten Werte eingestellt werden.

In Figur 3 sind ferner Verschlußmittel 60 auf der Innenseite eines Längsendabschnitts 62 des Längsabschnitts 6 dargestellt. Über diese Verschlußmittel können einander überlappende Längsendabschnitte 62 lösbar miteinander verbunden werden. Dies erweist sich insbesondere dann als vorteilhaft, wenn der gebrauchte Artikel durch die Öffnung 46 in das Aufnahmevolumen 44 des Entsorgungsbeutels eingebracht ist und der Beutel platzsparend verstaut werden soll, weil keine geeignete Entsorgungsmöglichkeit zur Verfügung steht. Der Hygieneartikel wird dann abermals um Querachsen gefaltet und durch die Verschlußmittel 60 in seiner gefalteten Konfiguration gehalten. Die Längsrandbereiche 36, 38 werden zwar dann abermals übereinandergefaltet, jedoch sind hier im allgemeinen keine wiederverschließbaren Verschlußmittel vorgesehen. Anstelle der dargestellten Verschlußmittel 60 in Form von Klebepunkten kann auch eine zusätzlich aufgebrachte Lasche mit einer Kleberbeschichtung vorgesehen sein.

Anhand der Figuren 4a bis 4d wird ein bevorzugtes Herstellverfahren des vorstehend beschriebenen Hygieneartikels mit Einzelumverpackung beschrieben. Die vorstehend mit dem Bezugszeichen 16, 18 beschriebenen Materialabschnitte, welche die schlauchförmige Bahn 4 bilden, werden zunächst in Form von endlosen Flachmaterialbahnen 16' und 18' in Längsrichtung 20 übereinander zugeführt. Es wird dann mittels nicht dargestellter Faltbleche die Z-förmige Faltung 28 in der oberen Flachmaterialbahn 16' eingebracht (Figur 4b).

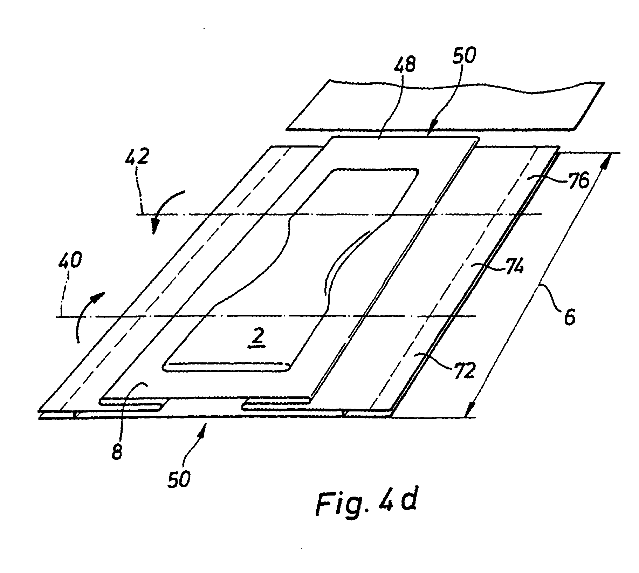

In einem weiteren Verfahrensschritt werden die Flachmaterialbahnen 16', 18' in Längsrichtung 20 und in einem Abstand A von den Längsrändern 22 unlösbar durch eine Siegelnaht miteinander verbunden, was durch die Verbindungslinie 24 dargestellt ist (Figur 4c). Es wird darauf hingewiesen, dass die äußere Falzlinie 32 einen größeren Abstand B von den Längsrändern 22 aufweist als die Verbindungslinie 24. Letztere ist daher in Querrichtung 26 außerhalb der äußeren Falzlinien 32 vorgesehen. Schließlich wird ein Hygieneartikel 2 auf die Innenseite 8 der oberen Flachmaterialbahn 16' aufgelegt und es wird von den Flachmaterialbahnen 16', 18' der in Figur 2 dargestellte Längsabschnitt 6 entlang der Trennlinie 50 abgetrennt. Ein Ende wird zusätzlich durch eine querverlaufende Siegelnaht 48 verschlossen (Figur 4d).

Nach Vereinzelung der Längsabschnitte 6 mit darauf aufliegendem Hygieneartikel 2 wird eine Faltung eines jeweiligen Längsabschnitts mit dem Hygieneartikel 2 um die Querachsen 40, 42 durchgeführt. Dabei wird ein erster Teilabschnitt 72 des Längsrandbereichs 36, 38 auf einen mittleren Teilabschnitt 74 des Längsrandbereichs 36, 38 gefaltet und ein dritter Teilabschnitt 76 des Längsrandbereichs 36, 38 wird auf den ersten Längsrandbereich 72 faltet. Es liegen somit im Überlappungsbereich der Längsrandbereiche insgesamt sechs Lagen der Materialabschnitte 16, 18 übereinander. Diese sechs Lagen werden durch Thermoprägen lösbar miteinander verbunden, so dass der Hygieneartikel samt Einzelumverpackung in der in Figur 3 dargestellten Konfiguration vor dem Gebrauch gehalten wird. Zum Entnehmen des Hygieneartikels, wird die lösbare Verbindung im Bereich der Teilabschnitte 72 bis 76 der Längsrandbereiche 36, 38 der jeweiligen Materialabschnitte 16, 18 gelöst und die Einzelumverpackung um die Querachsen 40, 42 aufgefaltet, so dass der Hygieneartikel 2 entnommen werden kann. Nach dem Gebrauch des Hygieneartikels kann dieser durch das offene Ende in das Aufnahmevolumen 44 des so gebildeten Entsorgungsbeutels eingegeben werden. Durch Auffalten der Z-förmigen Faltung 28 wird ein hinreichendes Aufnahmevolumen zur Verfügung gestellt.

## Patentansprüche

1. Hygieneartikel, wie Inkontinenzeinlage, Slipeinlage oder Damenbinde, mit einer Einzelumverpackung vor dem ersten Gebrauch, bestehend aus einem Längsabschnitt (6) einer schlauchförmigen eben gefalteten Bahn (4), mit einer eine Außenseite (12) und einer eine Innenseite (8) der Einzelumverpackung bildenden Flachseite (14 bzw. 10), und mit einer Längsrichtung (20), die parallel zu einer Längsachse des Hygieneartikels verläuft, wobei ein Längsende (62) des Längsabschnitts (6) flüssigkeitsdicht verschlossen und das andere Ende offen oder öffenbar ist, wobei aufeinandergefaltete Längsrandbereiche (36, 38) des Längsabschnitts (6) lösbar miteinander verbunden sind, um den Hygieneartikel in der Einzelumverpackung in gefalteter Konfiguration zu halten, unmittelbar vor dem Gebrauch aus der Einzelumverpackung herausnehmen und nach Gebrauch in das offene Ende des Längsabschnitts zum Entsorgen des Artikels einstecken zu können, **dadurch gekennzeichnet, daß** der Hygieneartikel in seiner gesamten Längserstreckung auf die die Innenseite (8) bildende Flachseite (10) des Längsabschnitts (6) der schlauchförmigen Bahn (4) aufgelegt ist und zusammen mit dem Längsabschnitt (6) um zwei senkrecht zu den Längsachsen verlaufende Querachsen (40, 42) auf sich selbst gefaltet ist.

2. Hygieneartikel mit einer Einzelumverpackung nach Anspruch 1, **dadurch gekennzeichnet, dass** der Längsabschnitt (6) der eben gefalteten schlauchförmigen Bahn (4) zwei übereinander angeordnete die Flachseiten (10, 12) bildende Materialabschnitte (16, 18) umfasst, die in einem Abstand (A) zu ihren jeweiligen Längsrändern (22) in Längsrichtung (20) flüssigkeitsdicht und unlösbar miteinander verbunden sind (24).

3. Hygieneartikel mit einer Einzelumverpackung nach Anspruch 1 oder 2, **dadurch gekennzeichnet, dass** die aufeinander gefalteten und lösbar miteinander verbundenen Längsrandbereiche (36, 38) des Längsabschnitts (6) in Querrichtung (26) außerhalb der unlösbaren Verbindung (24) der Materialabschnitte (16, 18) liegen.

4. Hygieneartikel mit einer Einzelumverpackung nach einem der vorstehenden Ansprüche, **dadurch gekennzeichnet, dass** die aufeinander gefalteten Längsrandbereiche (36, 38) des Längsabschnitts (6) durch Thermoprägung lösbar miteinander verbunden sind.

5. Hygieneartikel mit einer Einzelumverpackung nach einem oder mehreren der vorstehenden Ansprüche, **dadurch gekennzeichnet, dass** wenigstens einer der Materialabschnitte (16, 18) an wenigstens einer Längsseite wenigstens eine Z-förmige Faltung (28) mit einer inneren (30) und einer äußeren (32) in Längsrichtung verlaufenden Falzlinie aufweist.

6. Hygieneartikel mit einer Einzelumverpackung nach Anspruch 5, **dadurch gekennzeichnet, dass** die äußeren Falzlinien (32) in Querrichtung (26) innerhalb des Verlaufs der unlösbaren Verbindung (24) der Materialabschnitte (16, 18) miteinander angeordnet sind.

7. Hygieneartikel mit einer Einzelumverpackung nach einem oder mehreren der vorstehenden Ansprüche, **dadurch gekennzeichnet, dass** der Hygieneartikel an seiner körperabgewandten Außenseite (52), welche der die Innenseite (8) bildenden Flachseite (10) des Längsabschnitts (6) der schlauchförmigen Bahn (4) zugewandt ist, ein Haftklebemittel (34) zum Befestigen des Artikels am Steg eines Unterbekleidungsstücks aufweist.

8. Hygieneartikel mit einer Einzelumverpackung nach Anspruch 7, **dadurch gekennzeichnet, dass** der Hygieneartikel über das Haftklebemittel (54) direkt an der die Innenseite bildenden Flachseite anliegt und von dieser wieder lösbar ist.

9. Hygieneartikel mit einer Einzelumverpackung nach Anspruch 7, **dadurch gekennzeichnet, dass** das Haftklebemittel (54) über ein Ablösepapier (56) an die Außenseite (52) des Hygieneartikels appliziert ist und der Hygieneartikel unter Zwischenordnung des Ablösepapiers (56) gegen die die Innenseite (8) bildenden Flachseite (10) des Längsabschnitts (6) anliegt.

10. Hygieneartikel mit einer Einzelumverpackung nach Anspruch 9, **dadurch gekennzeichnet, dass** zwischen dem Ablösepapier (56) und der die Innenseite (8) bildenden Flachseite (10) ein vorzugsweise haftklebendes Fixiermittel (58) vorgesehen ist, um den Hygieneartikel zum Falten um die Querachsen (40, 42) in vorbestimmter Stellung zu halten.

11. Hygieneartikel mit einer Einzelumverpackung nach Anspruch 10, **dadurch gekennzeichnet, dass** die Haftkraftunterschiede des Ablösepapiers (56) am Hygieneartikel und an der Innenseite (8) so eingestellt sind, dass das Ablösepapier (56) beim Entnehmen des Hygieneartikels zum Gebrauch über das Fixiermittel (58) an der Einzelumverpackung verbleibt.

12. Hygieneartikel mit einer Einzelumverpackung nach einem oder mehreren der vorstehenden Ansprüche, **gekennzeichnet durch** ein vorzugsweise haftklebendes Verschlußmittel (60), welches die um die beiden Querachsen (40, 42) aufeinander gefalteten Längsenden (62) des Längsabschnitts (6) der schlauchförmigen Bahn (4) lösbar miteinander verbindet.

13. Verfahren zum Herstellen eines Hygieneartikels mit einer Einzelumverpackung nach einem oder mehreren der vorstehenden Ansprüche, **gekennzeichnet durch** folgende Merkmale:
Zuführen einer schlauchförmigen Bahn (4) oder einer ersten und einer zweiten Flachmaterialbahn (16' bzw. 18') zur Bildung einer schlauchförmigen Bahn (4) in eben gefalteter Konfiguration in einer Längsrichtung (20),
unlösbares Verbinden der eben gefalteten Abschnitte (16, 18) der schlauchförmigen Bahn (4) oder der ersten und der zweiten Flachmaterialbahn (16', 18') miteinander beidseits in Längsrichtung (20) und in einem Abstand (A) zu ihren jeweiligen Längsrändern (22),
aufeinanderfolgendes Zuführen von Hygieneartikeln (2) in deren Längsachsenrichtung parallel zur Längsrichtung (20) der Bahn und Auflegen auf die die Innenseite (8) bildende Flachseite (10) jeweils in Längsrichtung (20) beabstandet zueinander,
Trennen (50) der schlauchförmigen Bahn (4) quer zu ihrer Längsrichtung zwischen zwei aufgelegten Hygieneartikeln (2) zur Bildung von Längsabschnitten (6),
flüssigkeitsdichtes unlösbares Verbinden der eben gefalteten Abschnitte (16, 18) eines jeweiligen Längsabschnitts (6) in Querrichtung (48) zur Bildung eines verschlossenen Längsendes des jeweiligen Längsabschnitts (6) der schlauchförmigen Bahn (4),
Falten des Hygieneartikels (2) zusammen mit dem Längsabschnitt (6) der Bahn (4) um zwei senkrecht zur Längsachse verlaufende Querachsen (40, 42) auf sich selbst,
lösbares Verbinden von aufeinander gefalteten Längsrandbereichen (36, 38) des jeweiligen Längsabschnitts (6) der Bahn (4), um den Hygieneartikel (2) in der Umverpackung in gefalteter Konfiguration zu halten und zum Gebrauch aus der Einzelverpackung herausnehmen zu können.

14. Verfahren nach Anspruch 13, **dadurch gekennzeichnet, dass** die schlauchförmige Bahn (4) oder die erste und/oder die zweite Flachmaterialbahn (16', 18') beidseits um eine innere und eine äußere in Längsrichtung verlaufende Falzlinie (30, 32) Z-förmig gefaltet wird.

15. Verfahren nach Anspruch 14, **dadurch gekennzeichnet, dass** die Z-förmige Faltung (28) vor dem unlösbaren Verbinden der beiden Flachmaterialbahnen (16', 18') miteinander eingebracht wird.

16. Verfahren nach Anspruch 15, **dadurch gekennzeichnet, dass** die Z-förmige Faltung (28) derart eingebracht wird, dass die äußere Falzlinie (32) von den Längsrändern (22) der Flachmaterialbahnen (16', 18') nach innen beabstandet ist und auch innerhalb der unlösbaren flüssigkeitsdichten Verbindung (24) in Längsrichtung (20) verläuft, so dass sie beim unlösbaren Verbinden der aufeinander gefalteten Längsrandbereiche (36, 38) miteinander nicht erfasst wird.

## Revendications

1. Article d'hygiène, tel qu'une garniture pour incontinents, une garniture de slip ou une serviette hygiénique, qui comporte un emballage individuel à ouvrir avant le premier usage et constitué d'une section longitudinale (6) d'une bande (4) en forme de tuyau repliée à plat, une face plate (14), qui forme une face extérieure (12) de l'emballage individuel, et une face plate (10), qui forme une face intérieure (8) de l'emballage individuel, et une direction longitudinale (20) qui s'étend parallèlement à l'axe longitudinal de l'article d'hygiène, dans lequel une extrémité longitudinale (62) de la section longitudinale (6) est fermée de manière étanche aux liquides et l'autre extrémité est ouverte ou peut être ouverte, et dans lequel des zones de bord longitudinal (36, 38) de la section longitudinale (6), qui sont pliées l'une sur l'autre, sont reliées entre elles de manière détachable pour maintenir l'article d'hygiène en configuration repliée dans l'emballage individuel, le sortir de l'emballage individuel juste avant son usage et, après usage, pouvoir l'introduire dans l'extrémité ouverte de la section longitudinale (6) pour se débarrasser de l'article d'hygiène, **caractérisé en ce que** l'article d'hygiène est placé, dans toute son extension latérale, sur la face plate (10) de la section longitudinale (6) de la bande en forme de tuyau (4) qui forme la face intérieure (8) et est plié, avec la section longitudinale (6), sur lui-même autour de deux axes transversaux (40, 42) qui s'étendent perpendiculairement aux axes longitudinaux.

2. Article d'hygiène comportant un emballage individuel selon la revendication 1, **caractérisé en ce que** la section longitudinale (6) de la bande en forme de tuyau (4) pliée à plat comprend deux sections de matériau (16, 18) qui sont disposées l'une sur l'autre, qui forment les faces plates (10, 14) et qui sont reliées (24) entre elles de manière étanche aux liquides et de manière non détachable, dans la direction longitudinale (20), à une distance (A) de leurs bords longitudinaux respectifs (22).

3. Article d'hygiène comportant un emballage individuel selon la revendication 1 ou 2, **caractérisé en ce que** les zones de bord longitudinal (36, 38) de la section longitudinale (6), qui sont pliées l'une sur l'autre et qui sont reliées de manière détachable se trouvent, dans la direction transversale (26), à l'extérieur de la liaison non détachable (24) des sections de matériau (16, 18).

4. Article d'hygiène comportant un emballage individuel selon l'une quelconque des revendications précédentes, **caractérisé en ce que** les zones de bord longitudinal (36, 38) de la section longitudinale (6), qui sont pliées l'une sur l'autre, sont reliées entre elles de manière détachable par estampage à chaud.

5. Article d'hygiène comportant un emballage individuel selon l'une quelconque ou plusieurs des revendications précédentes, **caractérisé en ce qu'**au moins une des sections de matériau (16, 18) comporte, sur au moins une face longitudinale, au moins un pli en forme de « Z » (28) qui comporte une ligne de pliage intérieure (30) et une ligne de pliage extérieure (32) qui s'étendent dans la direction longitudinale (20).

6. Article d'hygiène comportant un emballage individuel selon la revendication 5, **caractérisé en ce que** les lignes de pliage extérieures (32) sont disposées, dans la direction transversale (26), à l'intérieur de l'extension de la liaison non détachable (24) des sections de matériau (16, 18).

7. Article d'hygiène comportant un emballage individuel selon l'une quelconque ou plusieurs des revendications précédentes, **caractérisé en ce que** l'article d'hygiène comporte, sur sa face extérieure (52), qui est opposée au corps et qui est dirigée vers la face plate (10) de la bande en forme de tuyau (4) qui constitue la face intérieure (8), un élément auto-adhésif (54) destiné à la fixation de l'article d'hygiène sur la bande d'une pièce de sous-vêtement.

8. Article d'hygiène comportant un emballage individuel selon la revendication 7, **caractérisé en ce que** l'article d'hygiène repose, par l'intermédiaire de l'élément auto-adhésif (54), directement sur la face plate (10) qui constitue la face intérieure (8) et peut être détaché de nouveau de cette dernière.

9. Article d'hygiène comportant un emballage individuel selon la revendication 7, **caractérisé en ce que** l'élément auto-adhésif (54) est appliqué sur la face extérieure (52) de l'article d'hygiène par l'intermédiaire d'un papier de détachement (56) et **en ce que** l'article d'hygiène est placé contre la face plate (10) de la section longitudinale (6) qui constitue la face intérieure (8) avec interposition du papier de détachement (56).

10. Article d'hygiène comportant un emballage individuel selon la revendication 9, **caractérisé en ce que**, entre le papier de détachement (56) et la face plate (10) qui constitue la face intérieure (8), on prévoit un moyen de fixation (58) qui est de préférence auto-adhésif pour maintenir l'article d'hygiène dans une position prédéterminée pour le plier autour des axes transversaux (40, 42).

11. Article d'hygiène comportant un emballage individuel selon la revendication 10, **caractérisé en ce que** les différences des forces d'adhérence du papier de détachement (56) sur l'article d'hygiène et sur la face intérieure (8) sont réglées de telle manière que, lorsque l'on enlève l'article d'hygiène pour son utilisation, le papier de détachement (56) reste sur l'emballage individuel par l'intermédiaire du moyen de fixation (58).

12. Article d'hygiène comportant un emballage individuel selon l'une quelconque ou plusieurs des revendications précédentes, **caractérisé par** un élément de fermeture (60) qui est de préférence auto-adhésif et qui relie entre elles de manière détachable les deux extrémités longitudinales (62) de la section longitudinale (6) de la bande en forme de tuyau (4) qui sont pliées l'une sur l'autre.

13. Procédé pour la fabrication d'un article d'hygiène comportant un emballage individuel selon l'une quelconque ou plusieurs des revendications précédentes, **caractérisé par** les caractéristiques suivantes :
amener une bande en forme de tuyau (4) ou une première et une deuxième bandes de matériau plat (16', resp. 18') dans une configuration pliée à plat dans une direction longitudinale (20) pour former une bande en forme de tuyau (4),
relier entre elles de manière non détachable les sections de matériau (16, 18), qui sont pliées à plat, de la bande en forme de tuyau (4) ou les première et deuxième bandes de matériau plat (16', resp. 18'), des deux côtés dans la direction longitudinale (20) et à une distance (A) de leurs bords longitudinaux respectifs (22),
amener successivement des articles d'hygiène (2) dans leur direction axiale longitudinale parallèlement à la direction longitudinale (20) de la bande (4) et les placer sur la face plate (10) qui constitue la face intérieure (8), à chaque fois dans la direction longitudinale (20) et avec une certaine distance entre eux,
séparer (50) la bande en forme de tuyau (4) selon une direction transversale par rapport à sa direction longitudinale, entre deux articles d'hygiène déposés pour former des sections longitudinales (6),
relier de manière non détachable et de manière étanche aux liquides des sections de matériau (16, 18) qui sont pliées à plat dans la direction transversale (48) pour former une extremite longitudinale fermée de chacune des sections longitudinales (6) de la bande en forme de tuyau (4),
plier l'article d'hygiène (2) avec la section longitudinale (6) de la bande en forme de tuyau (4) sur eux-mêmes autour de deux axes transversaux (40, 42) qui s'étendent perpendiculairement à l'axe longitudinal,
relier de manière détachable les zones de bord longitudinal (36, 38), qui sont repliées sur elles-mêmes, de chacune des sections longitudinales (6) de la bande en forme de tuyau (4) pour pouvoir conserver l'article d'hygiène (2) dans son emballage dans une configuration repliée et le sortir de son emballage individuel pour être utilisé.

14. Procédé selon la revendication 13, **caractérisé en ce que** la bande en forme de tuyau (4) ou les première et/ou deuxième bandes de matériau plat (16', resp. 18') sont pliées en forme de « Z » des deux côtés autour une ligne de pliage intérieure (30) et d'une ligne de pliage extérieure (32) qui s'étendent dans la direction longitudinale (20).

15. Procédé selon la revendication 14, **caractérisé en ce que** le pliage en forme de « Z » (28) est réalisé avant la réalisation de la liaison de manière non détachable des deux bandes de matériau plat (16', resp. 18') l'une avec l'autre.

16. Procédé selon la revendication 15, **caractérisé en ce que** le pliage en forme de « Z » (28) est réalisé de telle manière que la ligne de pliage extérieure (32) est à une certaine distance des bords longitudinaux (22), vers l'intérieur, et s'étend aussi dans la direction longitudinale (20) à l'intérieur de la liaison non détachable étanche aux liquides (24) de telle manière que cette dernière n'est pas saisie quand on réalise la liaison non détachable entre elles des zones de bord longitudinal (36, 38) qui sont repliées l'une sur l'autre.

## Claims

1. Sanitary article, such as an incontinence pad, panty liner or sanitary towel, with individual packaging prior to the first use, consisting of a longitudinal portion (6) of a tubular, planely folded web (4), with an outer side (12) and a flat side (14 and 10) forming an inner side (8) of the individual packaging, and with a longitudinal direction (20) extending parallel to a longitudinal axis of the sanitary article, one longitudinal end (62) of the longitudinal portion (6) being sealed in a liquid-tight manner and the other end being open or capable of being opened, longitudinal edge regions (36, 38) of the longitudinal portion (6) folded on top of one another being detachably connected to one another to hold the sanitary article in a folded configuration in the individual packaging, to be able to remove it from the individual packaging immediately prior to use and to be able to insert it into the open end of the longitudinal portion for disposal of the article after use, **characterised in that** the sanitary article is placed over its entire longitudinal extension on the flat side (10) of the longitudinal portion (6) of the tubular web (4) forming the inner side (8) and, together with the longitudinal portion (6), is folded onto itself about two transverse axes (40, 42) extending perpendicular to the longitudinal axes.

2. Sanitary article with individual packaging according to claim 1, **characterised in that** the longitudinal portion (6) of the planely folded tubular web (4) comprises two portions of material (16, 18) arranged one above the other and forming the flat sides (10, 14), the portions of material (16, 18) being connected to one another in a liquid-tight and non-detachable manner (24) at a distance (A) from their respective longitudinal edges (22) in the longitudinal direction (20).

3. Sanitary article with individual packaging according to claim 1 or 2, **characterised in that** the longitudinal edge regions (36, 38) of the longitudinal portion (6) folded on top of one another and detachably connected to one another are located outside of the non-detachable connection (24) of the portions of material (16, 18) in the transverse direction (26).

4. Sanitary article with individual packaging according to any of the preceding claims, **characterised in that** the longitudinal edge regions (36, 38) of the longitudinal portion (6) folded on top of one another are detachably connected to one another by thermostamping.

5. Sanitary article with individual packaging according to any one or more of the preceding claims, **characterised in that** at least one of the portions of material (16, 18) has at least one Z-shaped fold (28) on at least one longitudinal side, with an inner (30) and an outer (32) fold line extending in the longitudinal direction.

6. Sanitary article with individual packaging according to claim 5, **characterised in that** the outer fold lines (32) are arranged in the transverse direction (26) within the course of the non-detachable connection (24) of the portions of material (16, 18) to one another.

7. Sanitary article with individual packaging according to any one or more of the preceding claims, **characterised in that** on its body-remote outer side (52) facing the flat side (10) of the longitudinal portion (6) of the tubular web (4) forming the inner side (8), the sanitary article has an adhesive means (54) for fixing the article to the web of an article of underwear.

8. Sanitary article with individual packaging according to claim 7, **characterised in that**, via the adhesive means (54), the sanitary article is directly attached to the flat side forming the inner side and can be detached therefrom again.

9. Sanitary article with individual packaging according to claim 7, **characterised in that** the adhesive means (54) is applied to the outer side (52) of the sanitary article via a release paper (56) and the sanitary article rests against the flat side (10) of the longitudinal portion (6) forming the inner side (8) with interposition of the release paper (56).

10. Sanitary article with individual packaging according to claim 9, **characterised in that** a preferably adhesive fixing means (58) is provided between the release paper (56) and the flat side (10) forming the inner side (8) to hold the sanitary article in a predetermined position for folding about the transverse axes (40, 42).

11. Sanitary article with individual packaging according to claim 10, **characterised in that** the differences in the adhesion of the release paper (56) on the sanitary article and on the inner side (8) are adjusted such that the release paper (56) remains on the individual packaging via the fixing means (58) when the sanitary article is removed for use.

12. Sanitary article with individual packaging according to any one or more of the preceding claims, **characterised by** a preferably adhesive fastening means (60) which detachably connects to one another the longitudinal ends (62) of the longitudinal portion (6) of the tubular web (4) folded on top of one another about the two transverse axes (40, 42).

13. Method for producing a sanitary article with individual packaging according to any one or more of the preceding claims, **characterised by** the following features:
supplying a tubular web (4) or a first and a second flat material web (16' and 18') to form a tubular web (4) in a planely folded configuration in a longitudinal direction (20),
non-detachable connection of the planely folded portions (16, 18) of the tubular web (4) or the first and the second flat material web (16', 18') to one another on both sides in the longitudinal direction (20) and at a distance (A) from their respective longitudinal edges (22),
successive supplying of sanitary articles (2) in the direction of their longitudinal axis, parallel to the longitudinal direction (20) of the web and placement on the flat side (10) forming the inner side (8) at a distance from one another, in the longitudinal direction (20) in each case,
separating (50) the tubular web (4) transversely to its longitudinal direction between two positioned sanitary articles (2) to form longitudinal portions (6),
liquid-tight, non-detachable connection of the planely folded portions (16, 18) of a respective longitudinal portion (6) in the transverse direction (48) to form a closed longitudinal end of the respective longitudinal portion (6) of the tubular web (4),
folding the sanitary article (2), together with the longitudinal portion (6) of the web (4), onto itself about two transverse axes (40, 42) extending perpendicularly to the longitudinal axis,
detachable connection of longitudinal edge regions (36, 38) of the respective longitudinal portion (6) of the web (4) folded on top of one another to hold the sanitary article (2) in a folded configuration in the packaging and to be able to remove it from the individual packaging for use.

14. Method according to claim 13, **characterised in that** the tubular web (4) or the first and/or the second flat material web (16', 18') is folded on both sides in a Z-shaped manner about an inner and an outer fold line (30, 32) extending in the longitudinal direction.

15. Method according to claim 14, **characterised in that** the Z-shaped fold (28) is introduced prior to non-detachable connection of the two flat material webs (16', 18') to one another.

16. Method according to claim 15, **characterised in that** the Z-shaped fold (28) is introduced in such a way that the outer fold line (32) is at a distance inwardly from the longitudinal edges (22) of the flat material webs (16', 18') and also extends in the longitudinal direction (20) within the non-detachable liquid-tight connection (24), so it is not caught during non-detachable connection of the longitudinal edge regions (36, 38) folded on top of one another.
